(19) Europäisches Patentamt
European Patent Office
Office européen des brevets

(11) EP 4 686 936 A1

(12) EUROPEAN PATENT APPLICATION

(43) Date of publication:
**04.02.2026 Bulletin 2026/06**

(21) Application number: **24382832.4**

(22) Date of filing: **30.07.2024**

(51) International Patent Classification (IPC):
***G01N 27/327** (2006.01)* ***G01N 33/14** (2006.01)*

(52) Cooperative Patent Classification (CPC):
**G01N 27/3278; G01N 33/146**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Universitat Autònoma de Barcelona 08193 Bellaterra Barcelona (ES)**
- **Institut Catalá D´Investigació Química (ICIQ) 43007 Tarragona (ES)**

(72) Inventors:
- **Sala Román, Xavier 08193 Barcelona (ES)**
- **Francas Forcada, Laia 08193 Bellaterra (ES)**
- **Bofill Arasa, Roger 08193 Bellaterra (ES)**
- **Llobet i Dalmases, Antoni 43007 Tarragona (ES)**
- **Grau Abarca, Sergi 43007 Tarragona (ES)**
- **Gil Sepulcre, Marcos 43007 Tarragona (ES)**
- **Muñoz Martín, Jose 08193 Bellaterra (ES)**
- **Campos Lendínez, Ángel 08193 Bellaterra (ES)**
- **del Valle Zafra, Manel 08193 Bellaterra (ES)**
- **García-Antón Aviñó, Jordi 08193 Bellaterra (ES)**

(74) Representative: **Pons IP Glorieta Rubén Darío 4 28010 Madrid (ES)**

(54) **ETHANOL DETERMINATION IN ALCOHOLIC BEVERAGES USING AN OLIGOMERIC MOLECULAR CATALYST**

(57) The invention relates to a new use of an oligomeric Ru molecular catalyst for the determination of ethanol in alcoholic beverages, such as wine. It also relates to a method of ethanol determination. Said Ru complex allows to decrease the limit of detections to 0.01% v/v and to discriminate between methanol and ethanol.

EP 4 686 936 A1

## Description

The invention relates to a new use of an oligomeric molecular catalyst for the determination of ethanol in alcoholic beverages, such as wine. It also relates to a method of ethanol determination. Therefore, this invention has applications in the alcoholic beverage industry.

## BACKGROUND ART

Wine is a complex mixture of molecules originating from grapes and is an essential economic sector generating significant worldwide revenues. The quality and safety monitoring of wine production requires quantitatively determining a series of composition parameters, especially the concentration of alcoholic species.

The techniques for alcohol determination in wines range from basic to advanced methodologies. While advanced chromatographic methods like Gas Chromatography (GC) or High-Performance Liquid Chromatography (HPLC) offer high sensitivity with limit of detections (LODs) as low as 0.001 % v/v, they are quite tedious to be operated, requiring expensive equipment and skilled technicians to carry out the analytical measurements. In contrast, basic methods such as refractometry provide quicker results but with LODs typically at 0.1% v/v. This threshold presents a significant stumbling block, mainly when dealing with wines or alcoholic beverages bearing lower alcohol percentages, thereby inflating the analytical costs involved in their assessment.

Furthermore, the wine composition reveals that it comprises approximately 86% water and 12-13% alcohol, with the remaining fraction comprising a mixture of compounds that collectively define its flavour and character. Alcohol in wine is mainly represented by ethanol. Amidst these constituents, the potential presence of methanol, a toxic compound formed during fermentation, adds an extra layer of concern. Its consumption can precipitate severe health consequences, including irreversible blindness, organ failure, and even death when its presence goes undetected.

In order to overcome these limitations, electrochemical techniques open up new alternatives to develop rapid, low-cost and easy-to-automate analytical approaches by taking advantage of the electronic nature of the transduction method. This can promote decreasing LODs, enhancing sensibility and reaching high selectivity while avoiding the need to utilise costly benchtop techniques. Nonetheless, the LODs reported by electrochemical sensors so far do not fit the market's current demands, for example, reaching LODs as low as those obtained by advanced benchtop methods. In addition, the absence of readily available electrochemical sensors capable of effectively screening for methanol presence poses a significant hurdle. Albeit the versatility and applicability of electrochemical sensors for analysing a wide range of targets, the field of electrochemical sensors still struggles to discriminate methanol from the considerably higher ethanol background in alcoholic beverages, such as wine. Thus, the wine industry is compelled to continue its reliance on costly benchtop techniques, further amplifying the economic burden of addressing this critical concern.

Consequently, the present invention is focused on utilising a known oligomeric molecular catalyst as a highly sensitive moiety to be anchored on electrodes' surfaces to reach electrochemical sensors to detect ethanol in wine at LODs as close to the ones obtained by advanced benchtop techniques. In addition, the value added by the molecular catalyst allows the discrimination of ethanol from other alcoholic species (i.e., methanol). Importantly, this approach decreases costs and time while avoiding the need for trained personnel to carry out the measurements.

The oligomeric molecular catalyst used in the present invention has been previously described in Gil-Sepulcre, M. et al. J. Am. Chem. Soc. 2021, 143, 11651-1166111654, where it is used to oxidise water to dioxygen.

## SUMMARY OF THE INVENTION

The inventors of the present invention have found that the Ruthenium (Ru) complex, previously described in Gil-Sepulcre, M. et al. J. Am. Chem. Soc. 2021, 143, 11651-1166111654, can be used as an electrocatalyst or catalytically active material in the electrochemical determination of ethanol in alcoholic beverages. Surprisingly, said complex allows the decrease of the LODs to 0.01% v/v and to discriminate between methanol and ethanol, which implies a significant improvement over known methods and techniques for ethanol determination.

Then, the first aspect of the present invention refers to the use of a complex of formula (I):

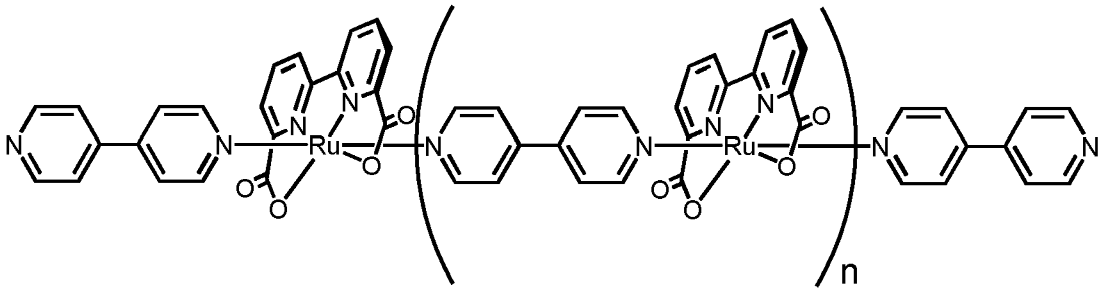

(I)

wherein n is an integer between 8 and 12,
for the electrochemical ethanol determination in alcoholic beverages.

Then, the Ru complex of formula (I) is an "oligomer", which refers to a molecule comprising a plurality of identical repeating units.

In a preferred embodiment of the use of the invention, n is 10.

The complex of formula (I) acts as an electrocatalyst (catalytically active material), which is usually anchored onto a carbonaceous solid material, forming an assembly that can be optionally deposited on solid support suitable for an electrode. Such an electrode may be incorporated into a device, such as an ethanol detection device. An ethanol detection device typically comprises three electrodes, a working electrode (WE), a counter electrode (CE) closing an electrical circuit, and a reference electrode (RE) in order to fix the working potential range.

Solid supports suitable for electrodes are known in the art and include conductive metal oxides, such as Indium tin oxide (ITO) or Fluorine-doped tin oxide (FTO), metals, alloys and glassy carbon. The support is further preferred to be a conductive metal oxide or glassy carbon.

Then, in a preferred embodiment of the use of the invention, the complex of formula (I) is anchored onto a carbonaceous solid material, preferably selected from graphite, graphene, reduced graphene oxide and carbon nanotubes (CNTs), more preferably, graphite or carbon nanotubes. The anchoring onto a carbonaceous solid material is through CH-$\pi$ interactions between the auxiliary ligands bonded to Ru and hexagonal rings of the graphitic surfaces, providing control of their molecular coverage.

In a preferred embodiment of the use of the invention, the alcoholic beverage is a beverage comprising ethanol in a concentration between 0.01 % and 20 % v/v.

In a more preferred embodiment of the use of the invention, the alcoholic beverage is wine.

The catalyst of formula (I) above-described can also be used to detect whether methanol is present in an alcoholic beverage.

Then, in a preferred embodiment, the catalyst of formula (I) is used for the electrochemical ethanol determination in alcoholic beverages and for detecting whether methanol is present in alcoholic beverages.

The catalyst of formula (I) has the ability to indicate the presence of methanol in a solution (alcoholic beverage) containing a mixture of methanol and ethanol. This is based on a shift in the electro-oxidation potential of + 15 to + 25 mV (preferably obtained *via Linear Sweep Voltammetry (LSV))* in a sample comprising both methanol and ethanol, compared to the electro-oxidation potential of pure ethanol (0.35 V $\pm$ 0.07 V vs. $Hg/Hg_2SO4$). This particularity makes it possible to identify adulterations in a beverage.

In a preferred embodiment, Linear Sweep Voltammetry (LSV) is used for the electrochemical ethanol determination in alcoholic beverages and/or for detecting whether methanol is present in alcoholic beverages.

As indicated above, the Ru oligomeric molecular catalyst of formula (I) allows not only the determination of ethanol in alcoholic beverages at very low concentrations but also the discrimination between methanol and ethanol. The outstanding performances using the complex of formula (I) can be attributed to the following benefits:

- In the first place, the interaction between the oligomeric molecular coordination complexes and the surface of carbonaceous materials, such as CNTs, is stronger than in the methods described in the art, which results in increased stability of the anchored catalyst and decreased risk of catalyst leaching.
- The provision of a compound with a plurality of catalytically active sites, or precursors thereof, which belong to the same molecular entity contributes to reaching high catalyst loadings onto the surface of the solid material, unlike the methods described in the art. This is thanks to the fact that, in the active compound, the catalytically active sites are close to each other. This spatial proximity of active sites is further translated onto the surface of the electrode once the compound is anchored on said surface.
- In addition, the provision of a ligand interacting with the surface of the solid material through non-covalent interactions

in such a manner that substantially all the active sites in the catalytically active materials are not facing the surface of the solid material is advantageous as it warrants that all the catalytically active metal atoms in the catalytically active material are accessible to the substrates of the reaction and are not buried within the supramolecular assembly consisting of the solid material and the catalytically active compound.

A second aspect of the invention refers to a method for the electrochemical ethanol determination in alcoholic beverages comprising the steps of:

(a) providing an electrode (WE) wherein the catalytically active material is the Ru molecular oligomer of formula (I) as defined in the first aspect of the invention,
(b) catalytic material activation by Cyclic Voltammetry (CV) means contacting the electrode (WE) of step (a) into a PBS buffer solution at pH higher than containing Pt wire (CE) while applying a potential between - 0.4 V and + 0.8 V vs $Hg/Hg_2SO_4$ (RE),
(c) contacting the electrode (WE) of step (b) with the alcoholic beverage while applying a potential between - 0.4 V and + 0.6 V vs. a reference electrode of $Hg/Hg_2SO_4$ (sufficient to promote the selective electro-oxidation of the ethanol at 0.35 V $\pm$ 0.07 V) and
d) obtaining the ethanol content by comparing the intensity obtained at a specific voltage in step c) (between - 0.4 V and + 0.6 V vs.) using a non-linear calibration curve.

The non-linear calibration curve is preferably obtained from different ethanol concentrations (from 0.01 % to 20 % v/v of ethanol), preferably by triplicate (n=5), *via* Linear Sweep Voltammetry (LSV), using a 1 M phosphate-buffered saline (PBS) solution as the electrolyte and contacting the electrode (WE) of step (b) with the solution while applying a potential between - 0.4 V and + 0.6 V vs. a reference electrode of $Hg/Hg_2SO4$.
In a preferred embodiment, the PBS buffer solution has a pH of 7.2 and a concentration of 1M.
In a preferred embodiment, the potential in step b) is applied over 50 cycles at a scan rate 0.1 $V/s^{-1}$.
The third aspect of the invention refers to a method for the electrochemical discrimination of adultered alcoholic drinks with other toxic alcohols (e.g. methanol) in alcoholic beverages, comprising the steps a) to c) described in the second aspect and, additionally, a step c)', between the steps b) and c) consisting of determining the potential of ethanol electro-oxidation *via Linear Sweep Voltammetry (LSV)* in samples containing ethanol as a unique alcohol *(from 0.01 % to 20 % v/v) in a 1 M phosphate-buffered saline (PBS) solution as the electrolyte* and *applying a potential between - 0.4 V and + 0.6 V vs. a reference electrode of $Hg/Hg_2SO4$ (Electro-oxidation of the ethanol may appear at 0.35 V $\pm$ 0.07 V);* and a step d), after step c), consisting of determining the potential at which electrooxidation is produced in step c), so that, if there is a shift of approximately from +15mV to +25 mV concerning the ethanol electro-oxidation determined in step c) (that may appear at 0.35 V $\pm$ 0.07 V), then it can be concluded that methanol is present in the alcoholic beverage.
Electro-oxidation of methanol is in the range 0.37 V $\pm$ 0.07 V as a consequence of the different energy required for their electro-oxidation,
**[0028]** This method allows for the screening and discrimination of adulterated samples with methanol.
In a preferred embodiment of the method of the invention, wherein the alcoholic beverage is wine.
As previously indicated, the use and method described in the present invention for the determination of ethanol in alcoholic beverages entail remarkable advantages over the prior art. In the quest to overcome the current limitations of electrochemical sensors in the field of alcoholic beverages (e.g., LODs in the range of 0.1% v/v and lack of discrimination with other alcoholic species like the hazardous methanol), the present invention provides a simple methodology based on the utilisation of oligomeric molecular catalysts as highly sensitive moieties to be anchored on electrodes' surfaces. This transformative shift from costly benchtop methods to low-cost electrochemical methods not only bolsters the economic viability of the wine production sector but also enhances safety measures and quality control, ultimately benefiting both producers and consumers alike. In an era of advancing technology and innovation, electrochemical techniques are poised to redefine and elevate the standards of the wine industry.
The consulted literature employs electrochemical techniques for the electrocatalysis of ethanol and methanol through systems involving metals, enzymes, and functionalised nanomaterials. The advantages of using oligomeric molecular catalysts compared to the prior state-of-the-art are varied.
The LOD values found in the prior art exceed those achieved in the system used for the invention, with most values being higher than 0.1% v/v for the detection of both ethanol and methanol and compromised ability to discriminate between ethanol and methanol. This is due to the similar oxidation potentials of both molecules, making their differentiation impossible in most cases. However, as demonstrated in the present invention (see examples), the oligomeric molecular catalyst (I) is capable of distinguishing between the redox potential of methanol and ethanol (0.37 $\pm$ 0.07 V vs 0.35 V $\pm$ 0.07 V, respectively), making their discrimination possible. Thus, implementing molecular catalysts allows for decreasing LODs at 0.01% v/v and discriminates between methanol and ethanol.
Finally, another advantage of the present invention is that the incorporation of the developed molecular catalyst

on screen-printed electrodes is feasible, making it possible for their direct integration on commercially available electrodes for their direct commercialisation.

Definitions

The term "catalytically active material" refers to a material suitable for promoting chemical reactions taking place at one or more active sites of the material. When the catalytically active material is also an electrocatalytically active material, said active sites are in contact with or comprised within an electrode and promote a chemical transformation involving the transfer of electrons between the electrode and the active sites.

The term "electrode" refers to an electrochemical transducer comprising a support suitable for an electrode comprising an electron conductive section, said support being used to close an electrical circuit through a medium, such as a solid or an ionic solution, separating two electrodes. An electrode suitable for electrocatalysis is an electrode further comprising an electrocatalytically active material that is used as a catalyst in an electrochemical reaction, such as a reduction or oxidation reaction.

The term "carbonaceous material" refers to a solid material composed essentially of carbon. Carbonaceous materials known in the art include, among others, graphite, carbon black, carbon felt, carbon paper, carbon nanotubes, graphene, reduced graphene oxide, and eventually doped with other atoms.

In the context of the invention, the term "determination" of ethanol refers to a quantitative detection of ethanol.

Unless otherwise defined, all technical and scientific terms used herein have the same meaning as commonly understood in the art to which this invention belongs. Methods and materials similar or equivalent to those described herein can be used in the practice of the present invention. Throughout the description and claims, the word "comprise" and its variations are not intended to exclude other technical features, additives, components, or steps. Additional objects, advantages, and features of the invention will become apparent to those skilled in the art upon examination of the description or may be learned by practising the invention. The following examples and drawings are provided by way of illustration and are not intended to limit the present invention.

**DESCRIPTION OF THE DRAWINGS**

**Fig. 1:** Cyclic Voltammetry (CV) activation spectra of Ru@CNTs samples (Ru catalyst of the present invention anchored onto CNTs) to activated $Ru(H_2O)_2$@CNTs over 50 cycles from - 0.4 V to + 0.8 V in a PBS buffer solution (pH ~7.2/ M = 1 M) at a scan rate of 0.1 $V/s^{-1}$. Samples were supported on a GC as a working electrode (WE) by a drop-casting of 30 μL each, using a platinum wire as a counter electrode (CE) and $Hg/Hg_2SO_4$ as a reference electrode (RE).

**Fig. 2:** Linear Sweep Voltammetry (LSV) spectra of a) CNTs and b) activated $Ru(H_2O)_2$@CNTs samples in the absence (solid line) and the presence (dashed line) of 1 % ethanol v/v in a PBS buffer solution (pH - 7.2/ M = 1 M). Samples were supported on a GC as a working electrode (WE) by a drop-casting of 30 μL each and activated to $Ru(H_2O)_2$@CNTs, using a platinum wire as a counter electrode (CE) and $Hg/Hg_2SO_4$ as a reference electrode (RE). Measurements were performed from 0 V to + 0.6 V for CNTs and from 0 V to + 0.6 V for Ru@CNTs potential at a scan rate of 0.1 $V/s^{-1}$.

**Fig. 3:** LSV spectra for a) ethanol and b) methanol determination (from 0.01% to 5%) using activated $Ru(H_2O)_2$@CNTs at different analytes volume range v/v concentrations in a PBS buffer solution (pH -7.2/ M = 1 M). Ru@CNTs samples were supported on a GC as a WE by a drop-casting of 30 μL each and activated to $Ru(H_2O)_2$@CNTs, using a platinum wire as a CE and $Hg/Hg_2SO_4$ as a RE. Samples were incubated for 5 min under stirring conditions (400 rpm) before each measurement. Experiments were performed from - 0.4 V to + 0.6 V potential at a scan rate of 0.1 $V/s^{-1}$. Non-linear calibration curves for c) ethanol and d) methanol were calculated from LSV spectra acquired using Eq. 1.

**Fig. 4**: LSV spectra for ethanol determination using activated $Ru(H_2O)_2$@CNTs at different analyte volume range v/v concentrations (from 1% to 20%) in a PBS buffer solution (pH -7.2/ M = 1 M). Ru@CNTs samples were supported on a GC as a WE by a drop-casting of 30 μL each and activated to $Ru(H_2O)_2$@CNTs, using a platinum wire as a CE and $Hg/Hg_2SO_4$ as a RE. Samples were incubated for 5 min under stirring conditions (400 rpm) before each measurement. Experiments were performed from -0.4 V to + 0.6 V potential at a scan rate of 0.1 $V/s^{-1}$. Non-linear calibration curve for ethanol was calculated from LSV spectra acquired using Eq. 1.

**Fig. 5**: Bar floating plot for methanol discrimination using different ratio complex mixtures (25:75, 50:50 and 75:25 of EtOH:MetOH) at different analyte volume range v/v concentrations (from 1% to 10%) in a PBS buffer solution (pH -7.2/ M = 1 M).

## Examples

Next, some examples carried out by the inventors are provided to illustrate the invention.

### Example 1: Preparation of the Ru catalyst of the present invention

The synthesis of the oligomeric molecular catalyst used in the present invention has been previously described in Gil-Sepulcre, M. et al. J. Am. Chem. Soc. 2021, 143, 11651-11661. A mixture of 50 mg (0.1 mmol) of [Ru(bda)$(dmso)_2$] and 15.6 mg (0.1 mmol) of 4,4'-bipyridine is placed in a 50 mL two-neck round bottom flask and degassed by vacuum-nitrogen cycles. Then, 10 mL of degassed TFE (2,2,2-trifluoroethanol) is added, and the resulting solution is heated under nitrogen at 78°C for 3 days. Afterwards, the solution containing the reaction mixture is evaporated to dryness and washed with degassed dichloromethane (DCM)/methanol (MetOH) (2:1) 5 x 5 mL. The remaining brownish solid is washed with degassed diethyl ether ($Et_2O$) and dried under vacuum, yielding Ru molecular oligomer as red-brown powder, stored under $N_2$.

In order to obtain different lengths of the catalyst, the ratios of ligand and precursor [Ru(bda)$(DMSO)_2$] as well as the organic solvents used could be changed. With the synthesis described above mostly oligomers with n=10 are obtained (as determined bu $^1$H-RMN).

### Example 2: Preparation of the Ru catalyst of the present invention anchored onto CNTs and the electrode comprising it

The functionalisation of CNT to form Ru@CNT is carried out by dissolving 1 mg of Ru molecular oligomer in 1 mL of pure TFE to form solution **A.** In parallel, solution **B** is prepared by sonicating 10 mg of CNT in 10 mL of pure THF for 20/40 minutes. Suspension **C** is prepared by mixing 0.1 mL of solution **A** and 1 mL of suspension **B.** The colour quickly disappears, indicating the anchoring of Ru molecular oligomer on CNT, resulting in Ru@CNTs.

### Example 3: Electrode activation

Ru@CNTs is sonicated for 10 minutes and carefully drop-cast 30 μL onto a glassy carbon GC (disk), resulting in the WE. Then, a three-electrode configuration cell filled with an electrolytic phosphate-buffered saline (PBS) solution (pH ~7.2/ M = 1 M) and a Pt wire as CE, $Hg/Hg_2SO_4$ as RE, and the WE containing the Ru@CNTs were immersed in the electrolytic solution. Subsequently, 50 oxidation and reduction cycles from - 0.4 V to + 0.8 V vs $Hg/Hg_2SO_4$ at 0.1 $V \cdot s^{-1}$ are performed, resulting in the activated $Ru(H_2O)_2$@CNTs with the inherent electrocatalytic ability to oxidise alcohols, as shown in Fig. 1.

### Example 4: Electrochemical measurements

Linear Sweep Voltammetry (LSV) measurements were carried out from - 0.4 V to 0.6 V at 0.1 $V \cdot s^{-1}$ scan rate, bringing different current intensities based on the EtOH concentration species in the media at 0.35 V. Fig.2 shows the current intensity change in the Linear Sweep Voltammetry (LSV) measurements of the CNTs (blank experiment) and the $Ru(H_2O)_2$@CNTs in the absence (0 %) and presence of 1 % *v/v* ethanol in a PBS solution (pH ~7.2/ M = 1 M). Herein, the Pt wire is used as CE, $Hg/Hg_2SO_4$ is used as RE, and a GC containing $Ru(H_2O)_2$@CNTs is used as the working electrode in a three-electrode configuration cell. Ethanol is added to the electrolytic solution, and the $Ru(H_2O)_2$@CNTs sample is incubated for 5 min under stirring conditions (400 rpm) before the spectra acquisition. Importantly, no current change was observed in the CNTs sample when exposed to ethanol species (Fig. 2a). In contrast, a significant increment in the current response was observed when the molecular catalyst was present on the CNTs surface (Fig. 2b), demonstrating the inherent catalytic response of these centres to oxidise alcoholic molecules in the media.

### Example 5: Sensing procedure

Once the catalytic response in the presence of ethanol was demonstrated, a sensing procedure was carried out using the activated $Ru(H_2O)_2$@CNTs samples in the presence of increasing alcohol concentrations (in v/v). Fig. 3a-b and Fig. 4a-b show the LSV spectra at different alcohol analytes (ethanol and methanol) at different volume rates (from 0.01 % -1 % v/v and from 1% to 20%). The measurements were carried out in an electrochemical cell filled with a PBS buffer solution (pH 7.2) while the sample was incubated for 5 min under stirring conditions (400 rpm) to favour catalyst-alcohol interactions. When the $Ru(H_2O)_2$@CNTs samples were exposed to ethanol, a new peak at E = + 350 mV appeared, which increased with increasing ethanol concentration (see Fig. 3a). As shown in Fig. 3c and Fig. 4c, regression curves were observed, yielding high sensibility, low error (n = 5) and excellent regression ($< r^2$: 0.99) in the measured range. Fig. 2c and

Fig. 3c were acquired using the Eq. 1 as follows:

$$y = \frac{I_f - I_0}{I_0} = \frac{\Delta I}{I_0} \qquad (1)$$

were $I_0$ is the maximum intensity current before and $I_f$ is the maximum intensity current after the ethanol incubation.

These results demonstrate the inherent electrocatalytic activity of the grafted molecular catalyst for the electro-oxidation of the ethanol substrate. In addition, the catalytic response could be achieved at high ethanol volume rates owing to the dense and high coverage achieved in the CNTs functionalisation from the oligomers containing the ruthenium centres.

On the other hand, the selectivity of the device was studied by carrying out the same experiment using methanol as the target. Fig. 3b and Fig. 4b show the voltammogram recorded for this analyte in the same conditions at volume ranges of 0.01 % to 1 % (Fig. 3b) and from 1 % to 20 % (Fig. 4b). As observed, a shift in the redox potential (E = + 370 mV) appears because of the different molecular nature of methanol compared to ethanol. This shift suggests that the discrimination between both alcohol structures is possible due to the different chemical nature (and oxidation potentials) of the two substrates. In addition, an excellent regression curve achieved using Eq. 1 can also be observed in Fig. 3d and Fig. 4d, demonstrating that the Ru@CNTs system can discriminate between these two alcohol molecules by means of redox potential.

All in all, the integration of oligomeric molecular catalysts on carbon-based electrodes yields the development of highly sensitive electrochemical sensors for monitoring oxidative reactions, as in the case of ethanol. In particular, the applicability of this strategy based on integrating oligomeric molecular catalysts on carbonaceous electrochemical transducers clearly overcomes the gap in the field of electrochemical sensors for monitoring alcoholic substances in alcoholic beverages. With this technology, highly sensitive, selective, low-cost and rapid electrochemical sensors have been achieved, improving LODs at levels of those obtained by advanced benchtop methods (< 0.01% v/v).

Additionally, in order to ensure the discrimination and the screening of ethanol and methanol in mixtures, different solutions comprising ethanol: methanol ratios of 75:25, 50:50 and 25:75 were prepared and exposed at different concentrations in the presence of $Ru(H_2O)_2$@CNTs. Fig. 5 shows a floating bar for different ethanol:methanol ratios (e.g., 100:0, 75:25, 50:50, 25:75, 0:100) electrooxidation potentials (mV) and no overlapped potentials with constant shifts of -+ 15 mV to + 25 mV with respect to the samples containing just ethanol were achieved, allowing to the screening discrimination of adultered mixtures with methanol.

## Claims

1. Use of a complex of formula (I):

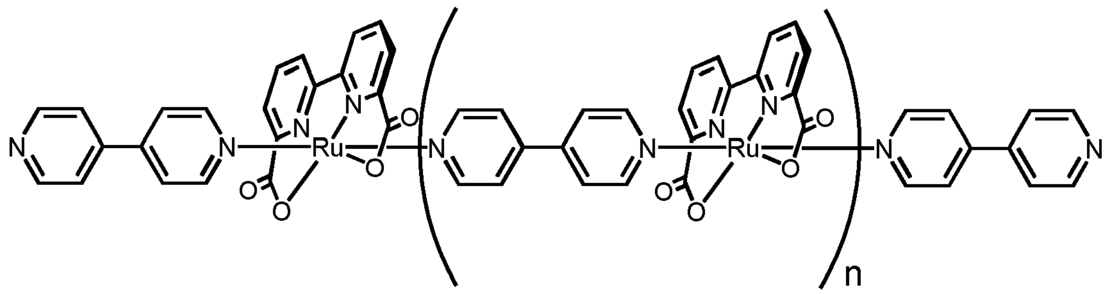

(I)

wherein n is an integer between 8 and 12
for the electrochemical determination of ethanol in alcoholic beverages

2. Use, according to any of the preceding claims, wherein the complex of formula (I) is anchored onto a carbonaceous solid material.

3. Use, according to claim 3, wherein the carbonaceous solid material is graphite or carbon nanotubes.

4. Use, according to claim 3 or 4, wherein the oligomeric complex of formula (I) anchored onto the carbonaceous solid

material is deposited on a solid support suitable for an electrode.

**5.** Use, according to claim 5, wherein the solid support suitable for an electrode is selected from the group consisting of glassy carbon and conductive metal oxides.

**6.** Use, according to any of the preceding claims, wherein the alcoholic beverage is wine.

**7.** Use of a complex of formula (I) as defined in claim 1, for detecting the presence of methanol in an alcoholic brevage.

**8.** Method for the electrochemical ethanol determination in alcoholic beverages comprising the steps of:

(a) providing an electrode (WE) wherein the catalytically active material is the Ru molecular oligomer of formula (I) as defined in the first aspect of the invention,
(b) catalytic material activation by Cyclic Voltammetry (CV) means contacting the electrode (WE) of step (a) into a PBS buffer solution at pH higher than containing Pt wire (CE) while applying a potential between - 0.4 V and + 0.8 V vs Hg/$Hg_2SO_4$ (RE),
(c) contacting the electrode (WE) of step (b) with the alcoholic beverage while applying a potential between - 0.4 V and + 0.6 V vs. a reference electrode of Hg/$Hg_2SO_4$ and
d) obtaining the ethanol content by comparing the intensity obtained at a specific voltage in step c) using a non-linear calibration curve.

**9.** Method for the electrochemical discrimination of adultered alcoholic beverages with methanol comprising the steps a) to c) described in claim 8 and, additionally, a step c)', between the steps b) and c), consisting of determining the potential of ethanol electro-oxidation *via Linear Sweep Voltammetry (LSV)* in samples containing ethanol as a unique alcohol *in a 1 M phosphate-buffered saline (PBS) solution as the electrolyte,* and *applying a potential between - 0.4 V and + 0.6 V vs. a reference electrode of Hg/$Hg_2SO_4$, and* a
step d'), after step c), consisting of determining the potential at which electro-oxidation is produced in step c), so that, if there is a shift of approximately from +15mV to +25 mV concerning the ethanol electro-oxidation determined in step c), then it can be concluded that methanol is present in the alcoholic beverage.

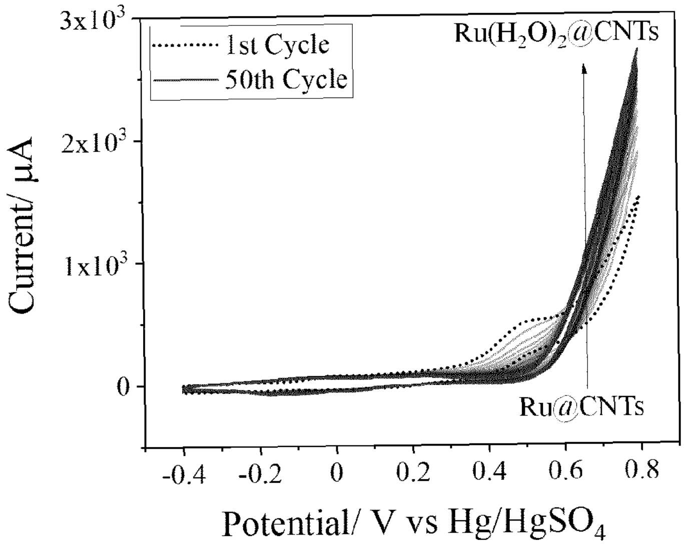

Current/ µA
Potential/ V vs Hg/HgSO4
1st Cycle
50th Cycle
Ru(H2O)2@CNTs
Ru@CNTs


Fig. 1

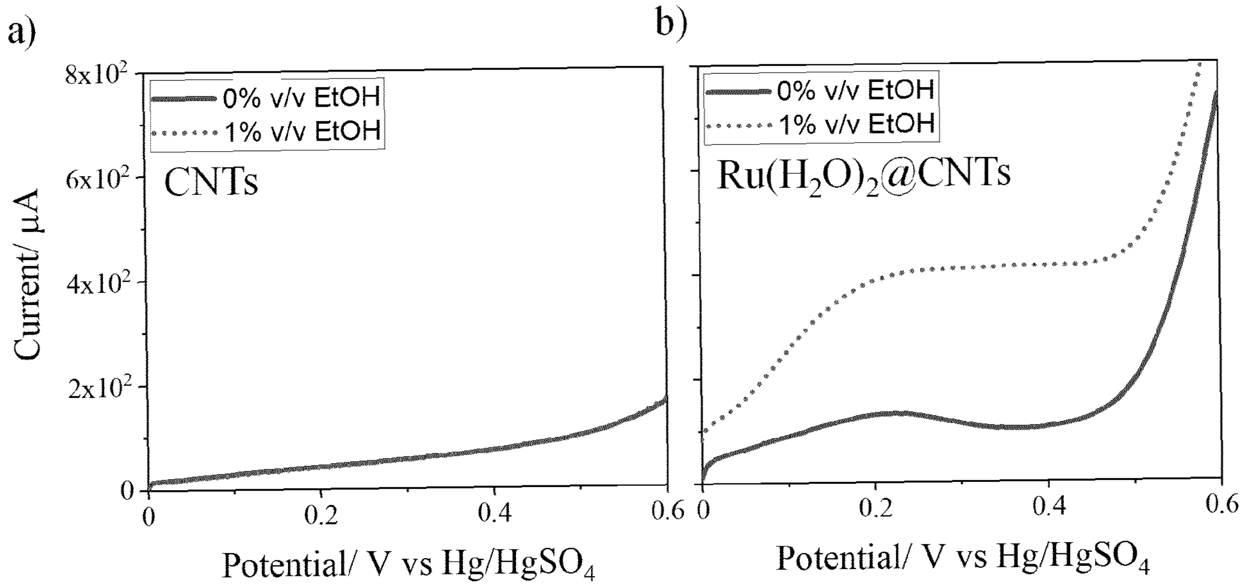

a)
b)
Current/ µA
0% v/v EtOH
1% v/v EtOH
CNTs
Ru(H2O)2@CNTs
Potential/ V vs Hg/HgSO4


Fig. 2

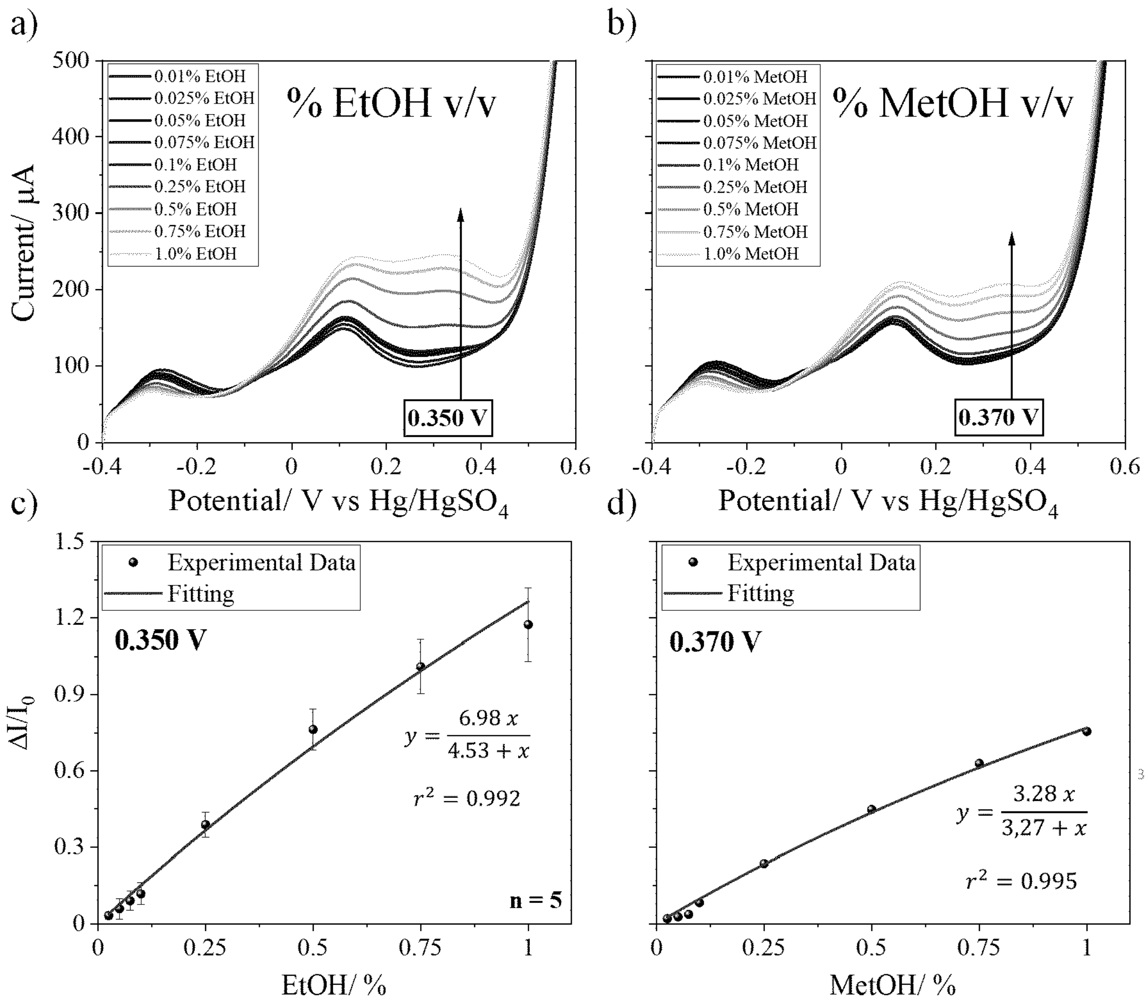
a)
b)
c)
d)
% EtOH v/v
% MetOH v/v
0.01% EtOH
0.025% EtOH
0.05% EtOH
0.075% EtOH
0.1% EtOH
0.25% EtOH
0.5% EtOH
0.75% EtOH
1.0% EtOH
0.01% MetOH
0.025% MetOH
0.05% MetOH
0.075% MetOH
0.1% MetOH
0.25% MetOH
0.5% MetOH
0.75% MetOH
1.0% MetOH
Current/ μA
Potential/ V vs Hg/HgSO4
0.350 V
0.370 V
Experimental Data
Fitting
ΔI/I0
y = 6.98 x / (4.53 + x)
r2 = 0.992
n = 5
y = 3.28 x / (3,27 + x)
r2 = 0.995
EtOH/ %
MetOH/ %

Fig. 3

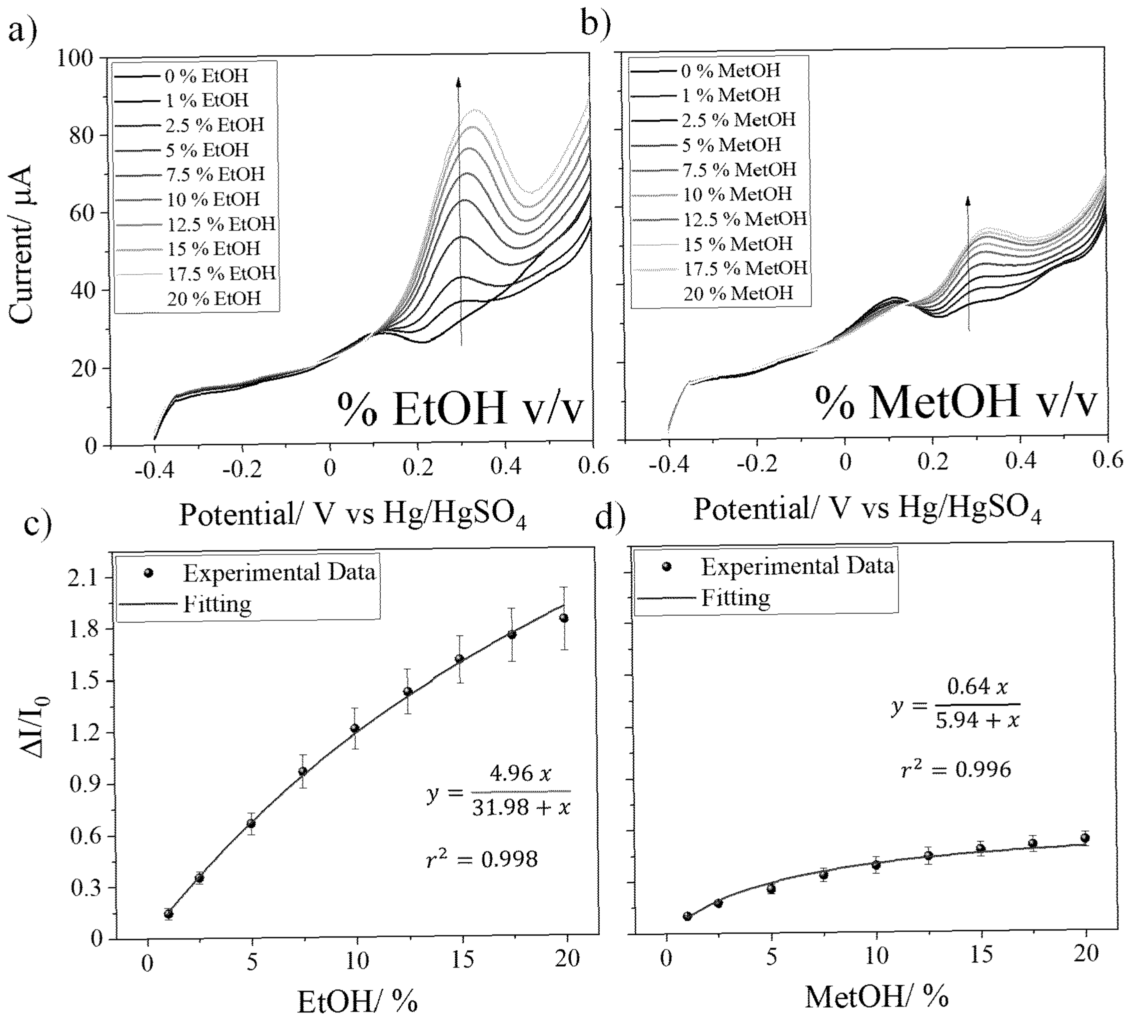

a)
b)
0 % EtOH
1 % EtOH
2.5 % EtOH
5 % EtOH
7.5 % EtOH
10 % EtOH
12.5 % EtOH
15 % EtOH
17.5 % EtOH
20 % EtOH
% EtOH v/v
Current/ µA
Potential/ V vs Hg/HgSO4
0 % MetOH
1 % MetOH
2.5 % MetOH
5 % MetOH
7.5 % MetOH
10 % MetOH
12.5 % MetOH
15 % MetOH
17.5 % MetOH
20 % MetOH
% MetOH v/v
Potential/ V vs Hg/HgSO4
c)
d)
Experimental Data
Fitting
ΔI/I0
y = 4.96 x / (31.98 + x)
r2 = 0.998
EtOH/ %
Experimental Data
Fitting
y = 0.64 x / (5.94 + x)
r2 = 0.996
MetOH/ %


Fig. 4

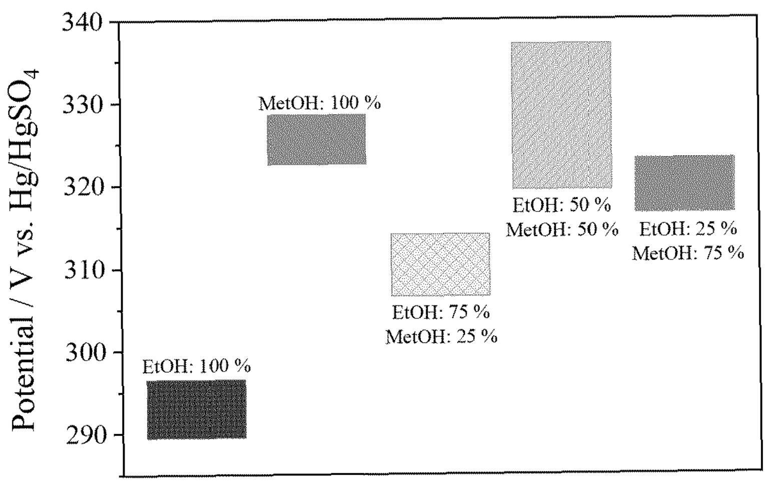

Potential / V vs. Hg/HgSO4
MetOH: 100 %
EtOH: 50 %
MetOH: 50 %
EtOH: 25 %
MetOH: 75 %
EtOH: 75 %
MetOH: 25 %
EtOH: 100 %


Fig. 5

Europäisches Patentamt
European Patent Office
Office européen des brevets

# EUROPEAN SEARCH REPORT

Application Number
EP 24 38 2832

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HOQUE MD ASMAUL ET AL: "Water oxidation electrocatalysis using ruthenium coordination oligomers adsorbed on multiwalled carbon nanotubes", NATURE CHEMISTRY, NATURE PUBLISHING GROUP UK, LONDON, vol. 12, no. 11, 28 September 2020 (2020-09-28), pages 1060-1066, XP037277856, ISSN: 1755-4330, DOI: 10.1038/S41557-020-0548-7 [retrieved on 2020-09-28] * abstract * * page 1060, left-hand column, paragraph 1 * * page 1061; figure 1 * ----- | 1-9 | INV. G01N27/327 G01N33/14 |
| A | EP 3 943 190 A1 (FUNDACIO PRIVADA INSTITUT CATALA DINVESTIGACIO QUIM ICIQ [ES]) 26 January 2022 (2022-01-26) * paragraphs [0013] - [0015] * * paragraph [0023] * * paragraphs [0046] - [0047] * ----- | 1-9 | TECHNICAL FIELDS SEARCHED (IPC) G01N |
| A | US 2011/311505 A1 (ERSOZ ARZU [TR] ET AL) 22 December 2011 (2011-12-22) * paragraphs [0253] - [0280] * * claims 35-38 * ----- | 1-9 | |

The present search report has been drawn up for all claims

1

| Place of search | Date of completion of the search | Examiner |
|---|---|---|
| Munich | 18 December 2024 | Michalitsch, Richard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 38 2832

This annex lists the patent family members relating to the patent documents cited in the above-mentioned European search report. The members are as contained in the European Patent Office EDP file on
The European Patent Office is in no way liable for these particulars which are merely given for the purpose of information.

18-12-2024

| Patent document cited in search report | Publication date | Patent family member(s) | Publication date |
|---|---|---|---|
| EP 3943190 A1 | 26-01-2022 | EP 3943190 A1 | 26-01-2022 |
| | | WO 2022018243 A1 | 27-01-2022 |
| US 2011311505 A1 | 22-12-2011 | US 2011311505 A1 | 22-12-2011 |
| | | WO 2011070402 A1 | 16-06-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **GIL-SEPULCRE, M. et al.** *J. Am. Chem. Soc.*, 2021, vol. 143, 11651-1166111654
- **GIL-SEPULCRE, M. et al.** *J. Am. Chem. Soc.*, 2021, vol. 143, 11651-11661